# EUROPEAN PATENT APPLICATION

(11) **EP 4 427 758 A1**
(43) Date of publication of application: **11.09.2024**
(21) Application number: 24161191.2
(22) Date of filing: 04.03.2024
(51) Int. Cl.: A61K 35/747, A61K 31/05, A61P 29/00, A61K 35/00

(54) **NEW SYNERGISTIC ANTI-INFLAMMATORY, ANTIOXIDANT AND ANTIBACTERIAL ASSOCIATION**

(30) Priority: 06.03.2023 IT 202300004017
(71) Applicant: NOOS S.r.l., 00181 Roma (RM) (IT)
(72) Inventor: Moretti, Mattia, 00128 Rome (IT); Mattioli, Roberto, 00166 Rome (IT); Di Risola, Daniel, 00166 Rome (IT); Mosca, Luciana, 00178 Roma (IT)
(74) Representative: Di Giovine, Paolo

(57) **Abstract**

The present invention relates to an association comprising a probiotic strain of the *Lactobacillus reuteri* species, both live and heat inactivated strain, and resveratrol (3,5,4'-trihydroxy-trans-stilbene), and in particular to the synergic effect shown by such association relatively to the antibacterial, antioxidant and anti-inflammatory activity with respect to the single components. The present invention, then, relates to an association between a probiotic strain of *L. reuteri* species and resveratrol, as well as to a pharmaceutical, cosmetic, nutraceutical composition, medical device, food supplement and a kit of parts comprising a strain of *L. reuteri* and resveratrol, and the use of the pharmaceutical composition, of the medical device or of the association, in a method of treatment in inflammatory pathologies.

## Description

### FIELD OF THE INVENTION

The present invention relates to an association comprising a probiotic strain of the *Lactobacillus reuteri* species, both live and heat inactivated strain, and resveratrol (3,5,4'-trihydroxy-trans-stilbene), and in particular to the synergic effect shown by such association relatively to the antibacterial, antioxidant and anti-inflammatory activity with respect to the single components. The present invention, then, relates to an association between a probiotic strain of the *L. reuteri* species and resveratrol, as well as to a pharmaceutical, cosmetic, nutraceutical composition, medical device, food supplement and a kit of parts comprising a strain of *L. reuteri* and resveratrol, and the use of the nutraceutical composition, of the medical device or of the association, in a method of treatment in inflammatory pathologies.

### STATE OF ART

Inflammation is an organism defence mechanism which activates in presence of damages to the tissues of various nature, including the one caused by pathogen agents (bacteria, viruses, parasites and fungi). It is a complex and essential process to counteract and neutralize the agent which caused it, to repair the produced damage and to restore the normal functions of the involved tissue or organ. It consists in a series of events which occur according to a precise sequence, in all, both external and internal, organic districts. The most common inflammatory forms appear at oro-intestinal and dermatological level and are commonly treated with antibiotics and anti-inflammatory drugs. These products, however, can cause side effects also of clinical relevance.

The inflammation treatment can differ based upon its gravity, the health conditions of the affected person and the presence of possible complications. Based upon the type of inflammation, the intensity of symptoms and the localization, two different types of anti-inflammatory drugs are available:
- *Nonsteroidal anti-inflammatory drugs* (FANS), which act by reducing the production of the molecules of the inflammatory process and can be used even to reduce pain and/or to lower the body temperature (antipyretics)
- *Cortisone or steroidal anti-inflammatory drugs,* which act by locking the action of substances used by the immune system to start the anti-inflammatory response or by interfering with the activity of the white blood cells.

### Contraindications and possible side effects

### FANS

The side effects of FANS are largely attributable to the inhibition induced by them of prostaglandin synthesis. It follows that the most common adverse reaction to the intake of FANS is the appearance of disorders affecting the digestive system, with the onset of nausea, vomiting, heartburn and diarrhoea. If use becomes habitual or prolonged, the formation of real ulcers of the gastrointestinal mucosa may occur with possibility of haemorrhages which could be mild but even, in particular cases, very serious. Therefore, the administration of FANS is not contraindicated only in cases of proven individual intolerance, but also in subjects suffering from previous or current gastro-duodenal ulcers, with previous digestive haemorrhages, in chronic liver or kidney diseases or at last in patients being treated with anticoagulants. Moreover, particular attention should be paid to the treatment of children, pregnant or breastfeeding women or the elderly.

At last, it should be noted that the ingestion of these drugs can cause the appearance of allergic skin rashes (itching, rash, urticaria), dizziness, drowsiness.

### Cortisone drugs

As far as the cortisone drugs are concerned, they can make people more vulnerable to infections. However, the extent of this and other risks depends upon various factors, in particular upon the type of taken corticosteroid, upon its dosage and the treatment duration. Among the various side effects hypertension, oedema, water retention, hypokalaemia, osteopenia, hyperglycaemia, insulin resistance, diabetes mellitus, muscle atrophy, delayed wound healing, redistribution of body fat with accumulation at the level of face, neck and abdomen, insomnia, increase in neutrophils and reduction in lymphocytes (anti-lymphoblastic action) are indicated.

The intake of oral corticosteroids for a long time involves the risk of developing diabetes, hypertension, glaucoma and cataract. The cortisone drugs, especially if taken long term, can trigger an increased appetite with consequent weight gain, thinning skin or acne appearance, peptic ulcers, slow healing of wounds and slow growth in children, muscle weakness or mood changes. The cortisone drugs which are inhaled, instead, can cause the appearance of thrush in the oral cavity, whereas the injections into the joints can cause pain and swelling. At last, injected in vein, the corticosteroids can cause tachycardia, insomnia and mood changes.

Moreover, the cortisone drugs can interact with other drugs or interfere with their action. Medicines at risk include anticoagulants, anticonvulsants, antidiabetics, antiretrovirals, bronchodilators, live vaccines and FANS.

### Antibiotics

For the treatment of pathological situations induced by bacterial pathogens with serious symptoms one has to recourse to the use of antibiotics, also with important side effects and induced resistance towards microorganisms.

The most frequent side effects are stomach pain, diarrhoea, general malaise, allergic reactions (especially to penicillins and cephalosporins, in rare cases a serious reaction (anaphylaxis) may occur which requires an urgent medical intervention. Moreover, some antibiotics are not suitable to be taken by people with determined diseases or by pregnant or breastfeeding women. For this, their use should occur with caution and only upon medical prescription.

### Bacteriotherapy

Bacteriotherapy represents an important therapeutic progress which with high safety profiles and by using lactobacilli and non-pathogenic bacteria is capable of affecting positively the genes performing an active role to improve some pathologies. It is a well-documented aid and therapeutic approach useful for improving the state of health and lifestyle of man for some chronic pathologies of gastrointestinal interest such as IBS, IBD, diverticular disease, as well as in the metabolic syndromes and in the cardiovascular diseases. The possibility of applying this method to the treatment of pathologies relating to different physiological districts is currently being evaluated.

### Probiotics at the dermis level

### Atopic dermatitis and intestinal bacterial flora

Scientific studies have detected that in the subjects with eczema or atopic dermatitis, the intestinal microbiota has significant differences compared to the microbiota of a healthy subject. It is clear that, even in this case, a good homeostasis and then the balance of the intestinal microbiota is fundamental to guarantee a general state of well-being.

The intestinal bacterial flora consists of numerous and different saprophytic bacterial strains and an unbalance of some species is sufficient to affect the organism health. The probiotics protect the surfaces of the epithelium and of the mucous membranes in the intestine by guaranteeing the correct operation thereof, thus preventing the adhesion and the invasion of the pathogens.

Evidence coming from different research groups show that the probiotics could have a positive effect on the treatment and care of the atopic dermatitis; the effect depends upon several factors, one thereof is the intake of specific strains. If then there was the possibility of a topical treatment, there would be the advantage of acting also at local level.

What illustrated above demonstrates that the treatment of pathologies and inflammatory situations could generate danger and it is necessary to find useful remedies, but as free as possible from side effects. The research in recent years has indicated for several substances interesting activities at anti-inflammatory level, but often insufficient to treat the different situations unless very high doses are used.

### SUMMARY OF THE INVENTION

The technical problem placed and solved by the present invention is to provide a natural product free of side effects useful for the treatment of inflammatory pathologies.

The Authors of the present invention have surprisingly demonstrated the synergy of the association between *L. reuteri,* in particular the LMG P-27481 strain, and resveratrol, on the antioxidant, anti-inflammatory and antibacterial activity with respect to the single substances.

The advantages of the association, the present invention relates to, are multiple. The main one consists in the synergy of the compound activity with respect to the effects of the single ingredients, with possibility of reducing the dosage of the same by obtaining the same, if not even better, activity.

In fact, a product constituted by the probiotic-resveratrol association has particular features given by the known effects of the single components. Specifically, resveratrol has a documented antioxidant, anti-inflammatory and antiviral effect whereas the strain of *L. reuteri* LMG-P-27481 has a marked anti-inflammatory and antibacterial activity as demonstrated in *in vitro* studies. The association of the two ingredients showed synergic effect on the examined parameters related to these conditions. For example, in Table 4 of Example 2 the activity of the *L. reuteri* and resveratrol association in a plate inhibition model is shown, showing that resveratrol contributes in the inhibiting activity of *L. reuteri* LMG P-27481 towards all pathogen strains, including *S. aureus,* for which K12 has no effect. The activity is comparable or higher than that of the *Streptococcus salivarius* K12, which is used in an oral spray product, currently market leader, designated as useful in the treatment of inflammations/infections of the oropharynx (including pharyngotonsillitis).

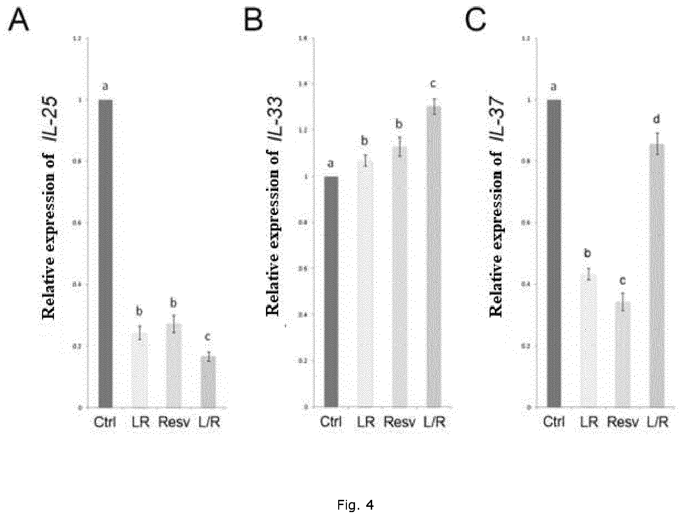

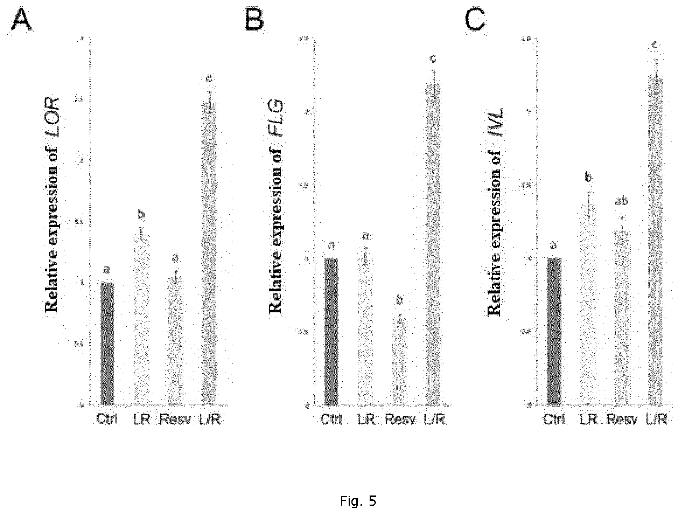

Additionally, in order to show the synergic activity of *L. reuteri* and resveratrol, Example 5 and Figures 4 and 5 show the evaluation of the gene expression of some cytokines (IL-25, IL-33, IL-37) and of filaggrin (FLG), involucrin (IVL) and loricrin (LOR) in a complex 3D model of *in vitro* reconstituted skin (EpiDermFT - MATTEK), whose results suggest a synergic action of resveratrol and *L. reuteri* in a complex model. In particular way, it seems that the resveratrol and *L. reuteri* combination, leads to a predominantly anti-inflammatory state and to the over-expression of epidermal integrity genes (LOR, FLG, IVL), responsible for the protection of the organ from external agents.

The association of a natural ingredient with a probiotic strain provided with particular anti-inflammatory and antibacterial features then represents a wished combination for the treatment of different inflammatory/bacterial/viral pathologies.

Moreover, the *L. reuteri,* in particular LMG P-27481, and resveratrol association allows to provide one single treatment for pathologies/situations which would require different pharmaceutical products with toxicity and multiple side effects, and capable of intervening on infections due to bacteria and viruses, as well as inflammations in general. It further allows a significant reduction in the use of antibiotics with lack of bacterial resistances induced by them, by representing an economically advantageous treatment and by reducing the expenses related to the complete treatment of infections that would require the use of multiple products.

Although probiotics are safe and usable even in critical situations, the possibility of using them after inactivation constitutes a very interesting therapeutic opportunity.

The inactivated bacteria are made uncapable of metabolizing and reproducing, their activity then cannot be traced back to the possibility of directly colonizing a physiological site. Their contribution constitutes a basis on which at intestinal level a substrate suitable for the recolonization of the symbiotic flora is reconstituted, by contrasting the development of pathogen strains. This is made possible by the macromolecules constituting the bacterial cell walls and those coming from cytoplasm which carry out a stimulating action towards the proliferation of the beneficial bacterial cells. At physiological level the same effects given by the use of live bacteria occur, such as the improved intestinal functionality, the stimulation of the immune system, the increase in the general state of well-being.

The heat inactivated strain additionally has advantages from the commercial point of view which reflect on the finished product given by the resveratrol-probiotic association. Its use is possible even in a medical device, where the use of live bacteria is not possible, there is no decay of the bacterial load in the finished product; there is the possibility of mixing with all the wished substances without any interference or reduction in the probiotic load; it is not necessary to maintain the cold chain and, at last, it is easy to formulate even in tablets.

Net of knowledge of the inventors, although there are other probiotics showing an antibacterial action towards pathogens, for no one an association with resveratrol nor with another anti-inflammatory active ingredient was studied.

Therefore, the present invention relates to:
• an association comprising a microorganism of the *Lactobacillus reuteri* species and resveratrol or a derivative thereof, wherein said derivative is methylated, hydroxylated, acetylated, glycosylated or halogenated. A not exhaustive list of the possible derivatives is shown in the following table:

| |
|---|
| Piceide |
| Pterostilbene |
| Trimethoxystilbene |
| Triacetyl-resveratrol |
| Tetramethoxysilbene |
| Pentamethoxystilbene |
| Dihydroxystilbene |
| Tetrahydroxystilbene |
| Hexahydroxystilbene |
| 2,6-dibromo-4-(3,5-dibromostyryl)phenol |
| 3,5-di-fluoro-4'-acetoxystilbene |
| 3,4,5-trimethoxy-4'-bromo-*cis*-stilbene |
| 4'-bromo-resveratrol |
| 2-bromo-resveratrol |
| 2-chloro-resveratrol |

• a composition comprising the association according to any one of the herein described embodiments, further comprising a pharmaceutically and/or nutraceutically acceptable excipient and/or carrier;
• a kit of parts comprising a microorganism of the *Lactobacillus reuteri* species as defined in any one of the herein described embodiments, and resveratrol or a derivative thereof as defined in any one of the herein described embodiments, wherein said derivative is methylated, hydroxylated, acetylated, glycosylated or halogenated;

Additional advantages and/or embodiments of the present invention will be evident from the following detailed description.

### DETAILED DESCRIPTION OF FIGURES

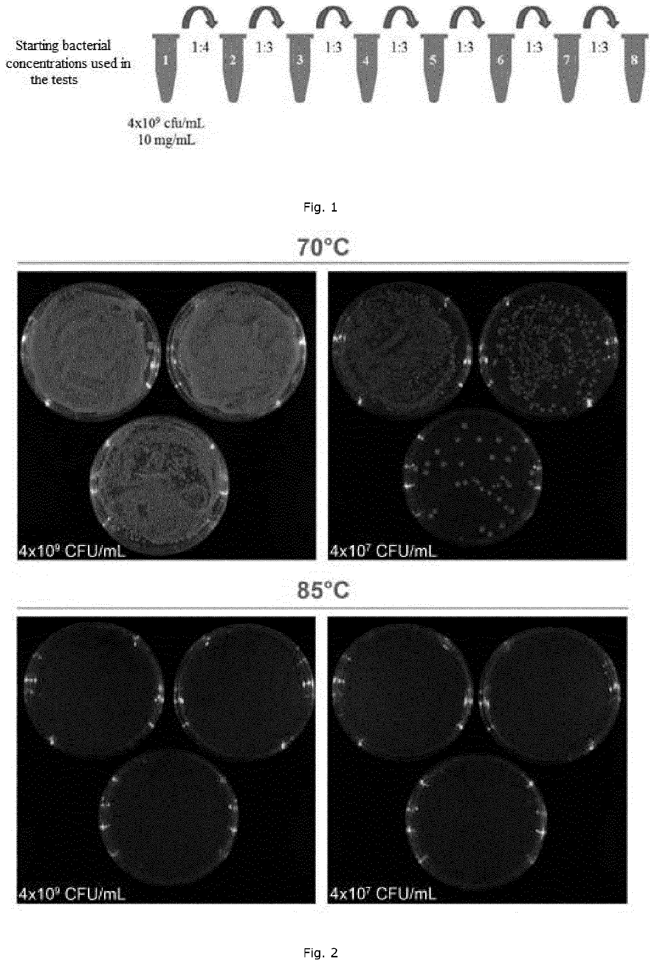

**Figure 1****.** Scheme of the 8 serial dilutions tested in the assays of antioxidant activity
**Figure 2****.** Petri Dishes for evaluating the bacterial count after heat inactivation for 15 minutes at 70°C and 85°C starting from two different concentrations of bacteria (4×10⁹ CFU/mL; 4×10⁷ CFU/mL)

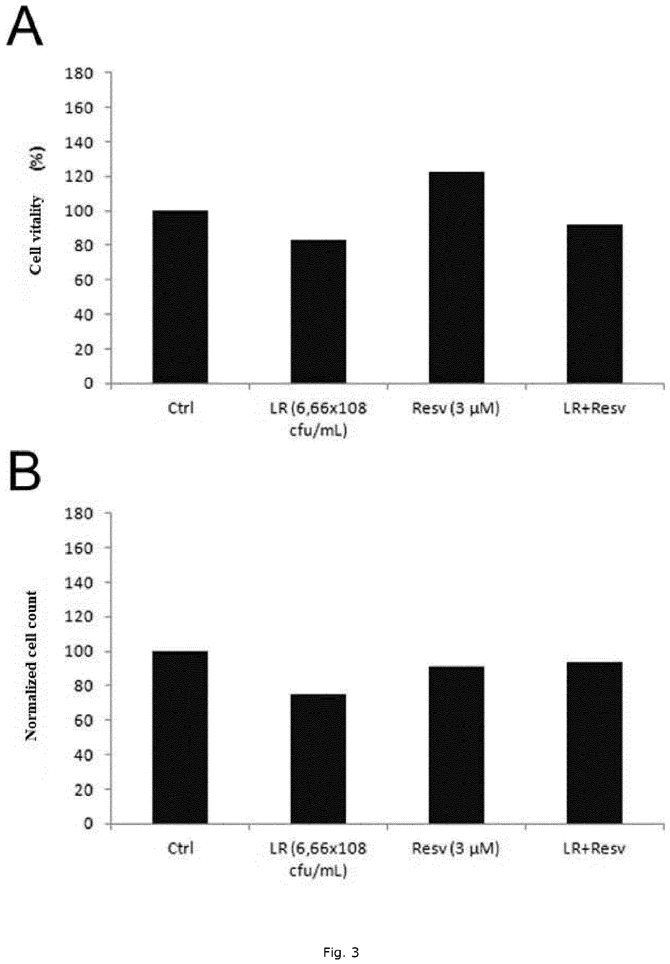

**Figure 3****.** MTT assay (cell viability) and crystal violet (cell count). A) MTT assay for determining the cell viability performed after 24 hours of treatment with *L. reuteri* (6.66×10⁸ CFU/mL), resveratrol (3 µM) and combination thereof; the values are normalized at 100 with respect to the control. B) crystal violet assay for determining the cell count performed after 24 hours of treatment with *L. reuteri* (6.66×10⁸ CFU/mL), resveratrol (3 µM) and combination thereof; the values are normalized at 100 with respect to the control.
**Figure 4** A) expression of **IL-25;** B) expression of **IL-33;** C) expression of **IL-37.** Quantification, by real-time PCR technique, of the expression of the genes under examination. The letters identify the group to which they belong from a statistical point of view. Different letters indicate statistical significance, equal letters indicate lack of statistical significance.
**Figure 5****.** A) expression of **LOR;** B) expression of **FLG;** C) expression of **IVL.** Quantification, by real-time PCR technique, of the expression of the genes under examination. The letters identify the group to which they belong from a statistical point of view. Different letters indicate statistical significance, equal letters indicate lack of statistical significance.

### GLOSSARY

### Resveratrol

Resveratrol (*trans*-3,5,4'-trihydroxystilbene) is a natural non-flavonoid polyphenol and it is produced by some plants in response to physiological stresses or bacterial and fungal infections. It works like a phytoalexin (a class of vegetable antibiotics) by protecting the plant from environmental stress or infections. It can be found in many types of fruits and vegetables and it is abundant in the grape peel. This molecule showed to be very useful for the human health and it has antioxidant, anticancer and antiviral activities, especially against the most common respiratory viruses, also having effect on diseases related to type 2 diabetes, cardiovascular diseases and neurological diseases.

Under derivative of resveratrol a compound with stilbene structure is meant, wherein the hydroxyl groups are placed in isomeric positions with respect to those of resveratrol, or are methylated, acetylated, glycosylated, or the stilbene skeleton is variously halogenated.

### Probiotics

According to the official definition of FAO and OMS, the probiotics are "live microorganisms which, administered in adequate amount, bring a benefit to the health of the host". They live naturally in the microbiota, by constituting approximately 15% of all the flora present in the intestine. Lactobacilli and bifidobacteria are the most common probiotic microorganisms.

The strains are not all the same, but a strain-specificity is known depending upon the immune, gene and anti-inflammatory features characterizing them.

The beneficial effects of the probiotics are known in many pathologies, such as IBD (Gut microbes 2012 jan 1; 3(1): 25-28). The activity mechanism includes the locking of the pathogen bacterial effects by raising the innate immunity, the decrease in the inflammation induced by the pathogens, the promotion of the survival of the epithelial intestinal cells which constitute the barrier function and are fundamental for the protective response.

### L. reuteri LMG P-27481 (Limosilactobacillus reuteri LMG P-27481)

From the Italian research a new strain of *Lactobacillus reuteri* was isolated, which was subjected to all studies provided by the International guidelines in order to be able to be defined probiotic. It was demonstrated that this strain has optimum probiotic properties, in terms of colonization, resistance to gastric acids and to bile salts, safety profile and stimulation of the immune system. Moreover, this strain, demonstrated important antimicrobial capabilities against the most common intestinal pathogens (ex. *E. coli, Salmonella,* Rotavirus) and more in particular against the pathogen *Clostridium difficile,* whose growth is strongly reduced, both in *in-vitro* tests and in experiments on animals. For these reasons the *Lactobacillus reuteri* LMG P-27481 strain presents itself as optimum candidate in the prevention and in the treatment of diarrheal disease especially the one induced by *C*. *difficile.* The *Lactobacillus reuteri* LMG P-27481 strain is provided with anti-inflammatory, immunomodulating activity and capability of inhibiting pathogen microorganisms and is set forth in the International patent application WO2019002914A1 incorporated herein in its entirety by reference, in particular in the portions which describe such microorganism.

### Pharyngitis

Pharyngitis, commonly called sore throat, is an inflammation of the mucosa coating the pharynx. It appears especially during the winter season, it affect both adults and children and very often it constitutes the beginning of an infection to the upper respiratory tract. It may appear suddenly and quickly (acute form) or persist over time (chronic form). Generally, the acute form is caused by an inflammation of viral or bacterial origin, it may associate to the presence of tonsillitis (pharyngotonsillitis) and can be caused even by inflammations which involve near organs (nose, paranasal sinuses, larynx). The chronic pharyngitis is frequently correlated with disease of other organs and apparatuses.

Pharyngitis can also be caused by general situations such as irritations caused by gastro-oesophageal reflux, abuse of smoking or alcohol, inhalation of irritating substances, pollution.

FANS are the products most commonly used for the treatment of this pathology, both in adults and in paediatric age. FANS act by inhibiting the synthesis of molecules involved in the inflammation (prostaglandins) by blocking the cyclooxygenase enzyme. This enzyme is constituted by different isoforms, the main ones are COX-1 and COX-2, which act at different levels, specifically COX-1 is a constitutive isoform normally expressed in the cells and involved in normal and physiological cellular processes. COX-2 is an inducible isoform, that is it is activated only after a tissue damage and it is responsible for the synthesis of pro-inflammatory prostaglandins. Most FANS on the market apart from inhibiting COX-2, also inhibits COX-1 and indeed the inhibition of COX-1 is at the origin of some of the unwished effects typical of all non selective FANS. Other FANS act selectively on COX-2, but however are not free of side effects of various kind.

When symptoms localized to the throat (pain, difficulty in swallowing, redness and itching of the throat mucosa) are prevailing, the topical formulations (oro-soluble tablets and spray) are the most suitable as they allow to carry the active ingredient directly in the inflamed area. In case of concomitant fever and infective complications it is better to have recourse to the administration by systemic route. In case of streptococcal pharyngitis it is necessary to have recourse to the antibiotic therapy which has to be prescribed by the doctor.

### Dermatological inflammations

### Acne

Acne is an inflammation of the pilosebaceous glands and appears with solid lesions, detected by the skin surface, which can be disfiguring, due to the action of germs. They can also be suppurated and, in the most serious cases, when the cysts join in the depth skin there is fistulisation.

The used treatments are given by pharmacological therapies (antibiotics, corticosteroids, retinoids, keratolytics, hormone therapy) which however are not conclusive and have side effects due to the nature of the drugs themselves. Even the dermo-aesthetic treatments can find application, but without solving the problem.

In some cases, especially when the cause which caused inflammation is difficult to be removed or a bacterial infection is present, pus may form (suppuration) which can accumulate inside a membranous capsule by causing an abscess. The abscess is difficult to treat and sometimes, in order to remove it, surgical incision is required.

### Atopic dermatitis

Atopic dermatitis (AD), also commonly called constitutional eczema, is a non-infective pathology of inflammatory type of allergic origin. AD is characterized by a sudden skin inflammation, which causes an annoying itching apart from a clearly visible redness. Usually it affects hands, feet, the inside crease of the elbow and the rear one of the knees, wrists, ankles, face, neck, chest, but manifestation around the eyes is also frequent.

The AD causes are not known. It is believed that there is also a hereditary component which induces a marked inflammatory reactivity towards common agents. The subjects suffering from AD have a defective skin barrier function, therefore they come into contact with substances normally kept outside. The currently used therapy aims at achieving a long-term stabilization, decreasing symptomatology and preventing flare-ups of the disease. The treatment of AD differs depending upon severity. Soothing self-medication products are available on the market, capable of relieving temporarily inflammation and itching. However, there are not products capable of definitively curing this inflammatory situation.

### DETAILED DESCRIPTION

The present invention relates to an association comprising a microorganism of the *Lactobacillus reuteri* species and resveratrol or a derivative thereof, wherein said derivative is methylated, hydroxylated, acetylated, glycosylated or halogenated.

In a preferred embodiment, said microorganism is the strain of *Lactobacillus reuteri* LMG P-27481 deposited at the BCCM/LMG Bacteria Collection microorganism collection center of the University of Ghent (Belgium).

In an embodiment, said microorganism, in particular said *L. reuteri* LMG P-27481, is a live or heat inactivated microorganism. The strain resuspended in physiological saline solution at a concentration equal to 4×10⁹ CFU/mL is treated for 15 min at 85°C, then it is centrifuged and the supernatant removed.

In an embodiment, the association of the invention comprises a microorganism of *L*. *reuteri,* preferably LMG P-27481 strain, and a plant extract comprising resveratrol. Preferably, the vegetable extract is an extract of *Polygonum cuspidatum,* of grapes, blueberry or peanuts.

The present invention further relates to a composition comprising the association according to any one of the herein described embodiments, and at least a pharmaceutically and/or nutraceutically acceptable excipient and/or carrier.

Pharmaceutically acceptable excipients and carriers suitable to the compositions of the present invention are known to the person skilled in the art depending upon the type of wished composition.

The association of the invention can be comprised in a pharmaceutical composition, a medical device, food supplement, functional food or a cosmetic product.

Medical device, in the present invention, corresponds to a medical device according to any of the classes described in the EU Regulation 2017/745 on the medical devices (which also comprises substances and not only "devices" in the mechanical sense of the term).

Under functional food, in the present invention, food is meant which, apart from the base nutritional properties, has a demonstrated capability to affect positively one or more physiological functions. Fundamental prerogative is also that of contributing to preserve or improve the state of health and/or to reduce the risk of onset of diet-related diseases.

In an embodiment, said microorganism is present in the composition, in food supplement, in the medical device, in the cosmetic product, in the pharmaceutical composition or in functional food of the invention in a concentration from 10² CFU/g to 10¹² CFU/g, preferably from 10⁶ CFU/g to 10¹⁰ CFU/g.

In an additional embodiment, said resveratrol is present in a concentration from 0.0001% to 50% w/v, preferably from 0.5% to 5% w/v, still more preferably 1% w/v.

In an embodiment, the composition, food supplement, the medical device, the cosmetic product, the pharmaceutical composition or functional food of the invention further comprises at least a stabilizing agent, preferably carboxymethyl beta-glucan (CMG) and/or deep eutectic solvents (DES - Deep Eutectic Solvents).

An example of formulation comprising the association relating to the invention is shown herebelow.

### Oral spray

It consists of *L.reuteri* LMG P-27481 in concentration comprised between 1×10⁶ and 1×10¹¹ CFU/ml and resveratrol 10 mg/ml (1% w/v), in oily suspension.

The present formulation can include even other ingredients useful for the well-being of the oral mucosa. The excipients of the present formulation are those normally used for the pharmaceutical and/or nutraceutical field.

The composition, food supplement, the medical device, the cosmetic product, the pharmaceutical composition or functional food of the invention can be administered by parental, oral, nasal, aerosol, sublingual, systemic, topical route. Additionally, the composition can be in form of suspension, emulsion, cream, spray, granulate, powder, solution, capsule, tablet.

In an embodiment, the association of the present invention, the composition, food supplement, the medical device, the cosmetic product, the pharmaceutical composition or functional food of the invention are for use as medicament. In a preferred embodiment, the association, the composition, food supplement, the medical device, the cosmetic product, the pharmaceutical composition or functional food in any of their embodiments, are for use in the prevention, in the treatment or in adjuvating the treatment of an inflammatory disease or an inflammatory state due to viral or bacterial infections.

According to an embodiment, the medical device of the invention is an oral spray, for use as adjuvant in the treatment of infections and/or inflammations of the oral cavity, or still a nasal spray for the treatment of infections and/or nasal inflammations and rhinitis, or still a cream for rectal use for the treatment of inflammations of the intestinal tract.

In an additional embodiment, a cosmetic product according to the present invention is a cream or an ointment for use in the treatment of dermatological pathologies.

In an embodiment, food supplement according to the present invention is a product to be taken orally or to be applied locally on the inflamed oral mucosa, having immunomodulating, anti-inflammatory, antibacterial and antiviral activity.

In particular, based upon the examples shown hereinafter, an unexpected synergy of the association between *L.reuteri,* in particular LMG P-27481 strain, and resveratrol, on the antioxidant, anti-inflammatory and antibacterial activity was demonstrated with respect to the single substances. This allows to provide one single treatment for pathologies/situations which would require different pharmaceutical products with toxicity and multiple side effects, and capable of intervening on infections due to bacteria and viruses, as well as to inflammations in general, and it allows a significant reduction in the use of antibiotics with lack of bacterial resistance induced by them.

In an embodiment, said inflammatory disease or inflammatory state is a dermatological inflammation, an inflammation of the gastrointestinal tract, an inflammation of the mucous membranes or an inflammation of the respiratory tract. In particular, said dermatological inflammation is selected from acne, rosacea, atopic dermatitis, contact dermatitis, seborrheic dermatitis, dermatitis eczematous, dermatitis herpetiformis, psoriasis said inflammation of the gastrointestinal tract is selected from ulcerative colitis, Crohn's disease, IBS (irritable bowel syndrome), IBD (inflammatory bowel disease), diverticular diseases, fissures, haemorrhoids, proctitis, said inflammation of the respiratory tract is selected from pharyngitis, tonsillitis, laryngitis, tracheitis, rhinitis, and said inflammation of the mucous membranes is selected from gingivitis, mouth ulcers, otitis.

A further object of the present invention is represented by a kit of parts comprising a microorganism of the *Lactobacillus reuteri* species as defined in any one of the herein described embodiments, and resveratrol or a derivative thereof as defined in any one of the herein described embodiments, wherein said derivative is methylated, hydroxylated, acetylated, glycosylated or halogenated.

In an embodiment, said kit allows a simultaneous administration or a sequential administration of the microorganism of the *Lactobacillus reuteri* species and of resveratrol.

In any part of the present description and claims, the term comprising can be replaced by the term "consisting of".

Examples are reported herebelow having the purpose of better illustrating the methodologies disclosed in the present description, such examples in no way are to be considered a limitation of the preceding description and of the subsequent claims.

### EXAMPLES

### Example 1

### Antimicrobial activity of the supernatant of Limosilactobacillus reuteri LMG P-27481, alone or in combination with resveratrol, against some selected pathogens (the supernatant from Streptococcus salivarius K12 was used as reference).

The present project studied the antimicrobial activity of the supernatant of *Limosilactobacillus reuteri* LMG P-27481, alone or in combination with resveratrol, towards five selected pathogens. The supernatant from *Streptococcus salivarius* K12 was used as reference. To this purpose, the Minimum Inhibitory Concentration (MIC) was evaluated. The pathogen microorganisms were the following ones: *Staphylococcus aureus* NCTC 6571, *Streptococcus pyogenes* LMG 21599, *Streptococcus pneumoniae* DSM 20566, *Moraxella chatarralis* DSM 9143, *Haemophilus influenzae* DSM 4690.

### Methods

### MIC determination

The Minimum Inhibitory Concentration (MIC) was evaluated by method of plate microdilution of supernatants of *L. reuteri* LMG P-27481 and *S. salivarius* K12, alone or in combination with resveratrol.

*L. reuteri* LMG P-27481 and *S. salivarius* K12 were subcultivated on agar plates from cryopreserved cultures. All pathogens, except for *H. influenzae* DSM 4690, were cultivated on agar BHI (Brain Heart Infusion, Oxoid). The subculture of *H. influenzae* DSM 4690 requested the presence of traces of lysed red blood cells in the medium.

In short, the supernatants were obtained for centrifugation of liquid cultures of *L. reuteri* LMG P27481 and *S. salivarius* K12, sterilized by filtration. The aliquots were diluted in series for determining the vital count. The supernatants were diluted in series from 100% to 0.19% (v:v).

The pathogen strains were dosed optically by using the spectrophotometer at OD600 between 0.08-0.100 and diluted 100 times in Schaedler broth or sterile supernatants (to obtain the concentration of 100%).

The tested concentrations of resveratrol varied from 640 to 1.25 µg/ml. All samples were incubated for 24 hours at 37°C under aerobic conditions for all strains except for *H*. *influenzae* DSM 4690 which required anaerobiosis conditions.

The MIC of the supernatant for *L.reuteri* LMG P-27481 was evaluated by serial dilutions whereas resveratrol was tested at one single dose obtained by preceding tests (data on file). Moreover, a control with serial dilutions of DMSO was performed in order to exclude that the presence of this diluent could exert an inhibitory effect on the pathogens.

### Results

Table 1 shows the data relating to MIC for *L. reuteri, S.salivarius* and resveratrol.

The values of MIC are expressed in percentage with respect to the dilution of the supernatant of the bacteria, whereas for resveratrol the data are shown in µg/ml.

**Table 1. Values of MIC obtained for L. reuteri LMG P-27481, S. salivarius K12, and resveratrol.**

| | **Supernatant LMG P-27481** | **Supernatant K12** | **Resveratrol** |
|---|---|---|---|
| | **(%)** | **(%)** | **(µg/ml)** |
| ***S*. *aureus*** | 50 | 100 | 160 |
| ***S*. *pyogenes*** | 25 | 100 | 160 |
| ***S*. *pneumoniae*** | 25 | 100 | 80 |
| ***M. chatarralis*** | 25 | 100 | 160 |
| ***H. influenzae*** | 12.5 | 100 | 80 |

The results highlight for *L.reuteri* LMG P-27481 a better activity than that of *S.salivarius* K12 which is usually used in a product useful for the treatment of the oropharyngeal infections. Resveratrol shows a MIC in line with the data reported in literature.

Table 2 shows the values of MIC determined for *L.reuteri* LMG P-27481 tested alone and in association with resveratrol. A significant synergic action for LMG P-27481 in combination with 40 µg/ml of resveratrol against *M. chatarralis* and *H. influenzae* is highlighted.

The values of MIC are expressed in percentage with respect to the dilution of the supernatant of bacteria.

**Table 2.**

| **Strains** | | **Supernatant** |
|---|---|---|
| | **Supernatant LMG P-27481** | **LMG P-27481 (%) + Resveratrol (40 µg/ml)** |
| | **(%)** | |
| ***S*. *aureus*** | **50** | **50** |
| ***S*. *pyogenes*** | **25** | **25** |
| ***S*. *pneumoniae*** | **25** | **25** |
| ***M. chatarralis*** | **25** | **1.56** |
| ***H. influenzae*** | **12.5** | **0.78** |

All tested pathogens were not inhibited by a high concentration of DMSO and then the inhibition of their growth has to be attributed exclusively to the contact with the tested strains and/or with resveratrol. The values of MIC of 100% for the supernatant *S. salivarius* K12 designate that the inhibition of the pathogens was reached at the maximum tested concentration, whereas for *L.reuteri* LMG P-27481 lower concentrations can be applied in order to obtain the inhibition of the growth of pathogens.

The data obtained for resveratrol comply with those reported in literature, with inhibiting concentrations for resveratrol varying from 80 to 160 µg/ml. Starting from these results for resveratrol, the concentration of 40 µg/ml was selected in order to exclude that resveratrol on itself could exert the inhibitory effect.

### Conclusions

The MIC values for all tested pathogens are comprised in the range of 80-160 µg/ml. When the resveratrol, at a lower concentration than MIC, was combined with the supernatant of *L. reuteri* LMG P-27481 determined a significant decrease in the MIC values against the Gram-negative pathogens. A significant synergic action in fact was found for LMG P-27481 in combination with 40 µg/ml of resveratrol against *M. chatarralis* and *H. influenzae.* The lack of synergy of the association towards the gram-positive pathogens is due to the used low concentration of resveratrol. Since they are very resistant bacteria, probably a higher concentration of resveratrol is required to obtain a decrease in MIC of the combination resveratrol with probiotic.

### Example 2

### Evaluation of sensitivity of Lactobacillus (Limosilactobacillus) reuteri LMG P-27481 to resveratrol under in vitro experimental conditions after the contact of 3×10⁸ CFU/ml of vital bacterium with four different final concentrations of resveratrol: 5 mg, 2.5 mg, 1 mg, and 0.5 mg per ml.

The test was performed by means of a plate inhibition model, by determining the growth of five pathogens of the upper airways: *Staphylococcus aureus* NCTC 6571, *Streptococcus pyogenes* LMG 21599, *Streptococcus pneumoniae* DSM 20566, *Moraxella chatarralis* DSM 9143 and *Haemophilus influenzae* DSM 4690.

In this second phase, the K12 strain of *Streptococcus salivarius* was included as control.

### METHODS

The simulated saliva (SS) was prepared with 8 g/l of sodium chloride, 0.19 g/l monobasic potassium phosphate, 2.38 g/l sodium phosphate (pH 6.8) and then sterilized (Alanzi et al., 2018).

The cryopreserve stock of *L. reuteri* LMG P-27481 strain was controlled in order to verify the purity thereof and then cultivated on MRS Broth (De Man Rogosa Sharpe, Difco) under microaerophilic conditions at 37°C for 18 hours.

The cultures in MRS broth of *L. reuteri* LMG P-27481 grown under microaerophilic conditions at 37°C for 18 hours were centrifuged, the supernatant discarded an the pellets were washed with sterile demineralized water. Concentrations of resveratrol of 5 - 2.5- 1 and 0.5 mg/ml were used for the tolerance test starting from a resveratrol mother solution of 50 mg/ml and then diluted to reach the final concentration for the test.

A test tube with only SS was used as positive control of survival.
*L. reuteri* LMG P-27481 was analysed according to the standard of Mc Farland in order to obtain a vital load of 3×10⁸ CFU/ml in the test tubes. 1 ml of SS suspensions and the scalar concentrations of resveratrol were put in contact and incubated at 37°C. Aliquots were collected after 5, 10, 30 and 60 minutes for determining vitality. All plates were incubated for 72 hours at 37°C under anaerobic conditions.

### Results

### Vitality of probiotics

- It is not affected by the presence of resveratrol at all considered dosages
- It is not affected by the presence of resveratrol after different contact periods of time (from 5 to 60 minutes)

**Table 3. Vitality of L.reuteri LMG P-27481 in presence of resveratrol**

| ***L. reuteri* (LMG P-27481)** | **%S*** | **%S*** | **%S*** | **%S*** |
|---|---|---|---|---|
| | **T5** | **T10** | **T30** | **T60** |
| Simulated saliva (SS) | 99.6 | 99.5 | 99.4 | 99.2 |
| SS + 5 mg/ml Resveratrol | 99.7 | 99.5 | 99.3 | 99.0 |
| SS + 2.5 mg/ml Resveratrol | 99.9 | 99.6 | 99.5 | 99.2 |
| SS + 1 mg/ml Resveratrol | 99.8 | 99.6 | 99.6 | 99.4 |
| SS + 0.5 mg/ml Resveratrol | 99.9 | 99.6 | 99.3 | 99.2 |

| | | | | |
|---|---|---|---|---|
| *%S = % of survival of microorganisms | | | | |

The vitality of *L. reuteri* LMG P-27481 is not influenced by the different tested concentrations of resveratrol. At the end of the test (T60), even at the maximum concentration of active ingredient (5 mg/ml), the survival rate is higher than 99%.

### Inhibition of pathogens

### L. reuteri LMG P-27481

- Effective towards all tested pathogens
- The association with resveratrol increases efficacy

### S. salivarius K12

- inhibition towards the tested pathogens lower than or equal to the one shown by L. reuteri LMG P-27481, except for *S*. *aureus* for which no activity is highlighted, not even in association with resveratrol.

**Table 4. Inhibition of pathogens**

| | LMG P-27481 | K12 | LMG P-27481 + Resv 5 mg/ml | K12 + Resv 5 mg/ml |
|---|---|---|---|---|
| *S*. *aureus* | ± | ------- (*) | +++ | ----- (*) |
| *S*. *pyogenes* | + | + | ++ | ++ |
| *S*. *pneumoniae* | ++ | + | +++ | ++ |
| *M. chatarralis* | ++ | ++ | +++ | ++ |
| *H. influenzae* | ++ | ++ | +++ | +++ |

| | | | | |
|---|---|---|---|---|
| (*) no inhibition | | | | |

### Conclusions

Resveratrol contributes to the inhibiting activity of *L.reuteri* LMG P-27481 towards all pathogen strains, including *S*. *aureus,* for which K12 has no effect. The activity is comparable to or higher than that of *Streptococcus salivarius* K12. This reference is particularly important since *Streptococcus salivarius* K12 is used in a oral spray product, currently market leader, designated as useful for contrasting the inflammations/infections of oropharynx (including pharyngotonsillitis).

### Example 3

### Determination of the in vitro antioxidant activity of Resveratrol and of L.reuteri LMG P-27481.

The in vitro antioxidant activity of resveratrol, of heat inactivated *Lactobacillus reuteri* LMG P-27481 and of their association was tested.

### Thermal inactivation of bacteria

For the inactivation of bacteria by heat, one started at a concentration of 4×10⁹ CFU/mL. The bacteria were then centrifuged at 4000 rpm for 10 minutes, the supernatant discarded and replaced with 1 ml of 0.9% NaCl saline solution. The heat inactivation occurred at the temperature of 85°C for 15 minutes. (Figure 2)

### NBT assay of antioxidant activity

NBT assay (nitrobluetetrazolium) evaluates the capability of locking the formation of the superoxide radical ^{·}O₂⁻; the more ^{·}O₂⁻ is produced, the more NBT is reduced in formazan. The capability of molecules and compounds to inhibit the NBT reduction by ^{·}O₂⁻ in presence of β-nicotinammide adenin dinucleotide (β-NADH) and phenazinamethosulfate (PM) is then tested.

In a quartz cuvette the following was inserted in the order: 838 µL of Tris HCl (0.02 M; pH 8.00), 20 µL of bacteria, of resveratrol or ascorbic acid at the different concentrations; 50 µL of NBT (1 mM), 77 µL of β-NADH (1 mM); 15 µL of PM (1 mM). The spectrophometric reading was performed by kinetics, by measuring the OD values (λₘₐₓ 560 nm) immediately and after 15 sec. The absorbance variation was used to calculate the inhibition percentage with respect to the control (solution of EtOH and of the solvent in which the different samples are dissolved).

The assays of antioxidant activity were performed on different bacterial concentrations and the activity curves were compared to those of molecules with known activity such as the ascorbic acid and resveratrol. Moreover, the bacterial concentrations having highest activity were tested in combination with resveratrol with the purpose of highlight a possible synergic, additive, null or inhibiting effect thereof.

To this purpose, on previously heat inactivated mixtures of bacteria, 8 serial dilutions were performed according to the scheme reported in figure 1; 20 µL of each dilution were then used in all assays of antioxidant activity according to the protocol indications.

Then, NBT assays were performed, wherein the capability of a molecule of operating as scavenging against the superoxide radical ^{·}O₂⁻ is tested. As shown in table 5, resveratrol seems to be the most effective one, with an IC₅₀ of 0.090 ± 0.012 mM, statistically lower than the IC₅₀ value shown by the ascorbic acid (0.665 ± 0.026 mM). Even the bacteria showed a good scavenging capability against the superoxide radical, by reaching inhibition percentages near to 100% for the highest concentrations; their IC₅₀ resulted to be 2.56×10⁷ ± 1.49×10⁶ CFU/mL. Moreover, the resveratrol + bacteria combination reduced IC₅₀ of resveratrol only, bringing it to 0.006 ± 0.002 mM and suggesting a synergic antioxidant capability.

These data show that the antioxidant capability shown by the bacteria increases when they are associated to resveratrol. The result further suggests that the heat inactivation protocol, despite having influenced bacterial vitality, did not compromise the activity of the antioxidant enzymes.

**Table 5. Antioxidant activity**

| Scavenging activity of the free radicals | IC₅₀ | | | |
|---|---|---|---|---|
| | Ascorbic Acid | Resveratrol (mM) | Bacteria (cfu/mL) | Resv.+Bacteria |
| | (mM) | | | (mM) |
| NBT Test (O2^{-•}) | 0.665 ± 0.026 | 0.090 ± 0.012** | 2.56·10⁷± 1.49·10⁶ | 0.006 ± 0.002^{#} [N.B.] |

| | | | | |
|---|---|---|---|---|
| ** p-value < 0.01 vs Ac.Asc. # p-value < 0.05 vs Resv. N.B.: combination implemented with 20 µL of a solution of bacteria 1×10⁹ CFU/mL | | | | |

### Example 4

### Determination of the maximum amount of resveratrol to be used by verifying if the association with inactivated probiotic interferes on cell viability

### Determination of cell viability by MTT assay and crystal violet

### Check on HaCat. cells

The test aims at evaluating the impact of the different substances on cell viability, in order to ensure the absence of negative effects or to establish the optimum concentrations of substances to be administered so that such effects do not appear.

### Methods

MTT test is a colorimetric assay which exploits the capability of cells to convert, at mitochondrial level, the bromide of 3-(4,5-dimethylthiazol-2-il)-2,5-diphenyltetrazolium in the related salt, by evaluating the cell viability. The produced salt is solubilized in organic solvent in order to obtain a homogeneous solution in the single well, measurable spectrophotometrically. Besides, the crystal violet assay is based upon the capability of the homonymous acidophilic dye to bind to DNA of the cells in culture and to return in this way a reliable estimation of the number of cells. Before proceeding with the assays, the cells were put in culture and treated for 24 or 48 hours with bacteria (at different concentrations), resveratrol (at different concentrations) and combinations thereof. Before colouring, washings in PBS were carried out. 50 µL of dye (MTT (5 mg/mL in PBS); Crystal violet (2,5 mg/mL in MeOH 20%)) were then added to each well and left in incubation at 37°C for 2 hours (MTT) or stirred on tilting plate for 20 min (Crystal violet). The exceeding dye was washed and the dye was solubilized in organic solvent (DMSO) in order to obtain a homogenous solution in the single well, measurable spectrophotometrically. The measurement of absorbance of the single wells was performed in a plate reader, at the wavelength of 570 nm, by normalizing the values with respect to the control.

### Results

The combination of the two tests (MTT and crystal violet) allowed to determining the maximum concentration of bacteria and resveratrol to be administered to the cells without these impacting on the count and on the cell viability (Figure 3).

Both MTT test and the analysis through crystal violet highlighted a decrease in vitality and cell count depending upon the concentration of resveratrol and the bacterial one. In particular, the higher the concentration of resveratrol or that of bacteria is, the higher the decrease in vitality is. However, the combination of resveratrol and bacteria in determined ratios seems to bring the vitality and the cell count near to the control one.

Based upon these results, the concentrations of resveratrol and heat inactivated *Lactobacillus reuteri* were determined, to be used in the experiments for determining the anti-inflammatory activity (3 µM of resveratrol; 6.66×10⁸ CFU/mL of bacteria)

### Example 5

### Evaluation of the gene expression of some cytokines (IL-25, IL-33, IL-37) and of filaggrin (FLG), involucrin (IVL) and loricrin (LOR) in a complex 3D model of in vitro reconstituted skin (EpiDermFT - MATTEK).

The 3D models of *in vitro* reconstituted skin constitute optimum systems capable of simulating well the functionalities of a complex organ such as skin and the interactions which are established between numerous cell populations composing it. Then, they try to free from the excessive simplification represented by one single cell line put in culture. The cytokines IL-25 and IL-33 are pro-inflammatory cytokines over-produced by the keratinocytes and by the surface layers of the skin after an insult; on the contrary the cytokine IL-37 is an anti-inflammatory cytokine. At last, the proteins filaggrin (FLG), involucrin (IVL) and loricrin (LOR), are proteins produced by epidermis, fundamental in the constitution of the barrier which protects from external agents and respond to cytokine signals. In particular, during an inflammatory event, the expression of these genes is sub-regulated; on the contrary, an anti-inflammatory state tends to over-express the genes FLG, IVL, LOR.

### Methods

### Real-time PCR

The extraction of total RNA was performed by QlAzol Lysis Reagent by following the instructions provided by the firm (Qiagen). The retro-transcription reactions were performed on 4 µg of total RNA by following the instructions provided by the protocol of the kit PROMEGA "GoTaq 2 Step RT qPCR System". The amplification for determining the gene expression, by real-time PCR technique, was performed on BioRad CFX96 machine and the related quantification was performed by the method of 2^{-ΔΔCt}.

### Results

For the IL-25 gene (Figure 4A), the treatment with bacteria causes a gene under-expression by demonstrating an anti-inflammatory action of the treatment. Similar results are obtained after the treatment with resveratrol only. The interesting thing is that the combination of resveratrol and *L. reuteri* produces a marked gene under-expression, statistically different from all other samples, by suggesting a synergic action of resveratrol and *L. reuteri.* As far as the IL-33 gene expression is concerned, a slight over-expression for all treatment groups with respect to control is registered (Figure 4B). This piece of data should not be read alone, but in the general picture of "panel" of expression of different cytokines. In particular, while for the IL-25 gene a strong under-expression is registered, there is a slight positive modulation of the gene of IL-33 cytokine which, probably, constitutes a response to feedback of the first one. The gene of the IL-37 anti-inflammatory cytokine, after 24 hours, registers its under-expression in the groups resveratrol and *L. reuteri.* On the contrary, in the co-treatment group, the expression levels tend to those of the control. Even in this case a synergy between resveratrol and *L. reuteri* is suggested (Figure 4C).

As far as the genes of the epidermic integrity (LOR, FLG, IVL) are concerned (Figure 5), the treatment with resveratrol and *L. reuteri* administered singularly lead to minimum expression variations; on the contrary, the treatment with the combination of resveratrol and *L. reuteri,* causes the LOR, FLG, IVL genes to be over-expressed significantly with respect to all other groups, by suggesting once again a synergic action of resveratrol and *L. reuteri.*

### Conclusions

These results suggest a synergic action of resveratrol and of the *L. reuteri* LMG P-27481 strain on a complex 3D model of *in vitro* reconstituted skin. Particularly, it seems that the combination resveratrol and *L. reuteri* leads to a mainly anti-inflammatory state and to the over-expression of the genes of epidermic integrity (LOR, FLG, IVL), responsible for the organ protection from external agents.

A formulation example comprising the association relating to the invention is reported hereinafter.

### Oral spray

It consists of *L.reuteri* LMG P-27481 in concentration comprised between 1×10⁶ and 1×10¹¹ CFU/ml and resveratrol 10 mg/ml , in oily suspension.

The present formulation can include even other ingredients useful for the well-being of the oral mucosa. The excipients of the present formulation are those usually used for the pharmaceutical/nutraceutical field.

## Claims

1. An association comprising a microorganism of the *Lactobacillus reuteri* species, and resveratrol or a derivative thereof, for use in the treatment or in adjuvating the treatment of a dermatological inflammation, an inflammation of the mucous membranes or an inflammation of the respiratory tract or an inflammation of the gastrointestinal tract selected from fissures, haemorrhoids, proctitis, and wherein said derivative of resveratrol is selected from Piceid, Pterostilbene, Trimethoxystilbene, Triacetyl-resveratrol, Tetramethoxysilbene, Pentamethoxystilbene, Dihydroxysilbene, Tetrahydroxystilbene, Hexahydroxystilbene, 2,6-dibromo-4-(3,5-dibromostyryl)phenol, 3,5-di-fluoro-4'acetoxystilbene, 3,4,5-trimethoxy-4'-bromo-cis-stilbene, 4'-bromo-resveratrol, 2-bromoresveratrol or 2-chloro-resveratrol.

2. The association according to claim 1, wherein said dermatological inflammation is selected from acne, rosacea, atopic dermatitis, contact dermatitis, seborrheic dermatitis, dermatitis eczematous, dermatitis herpetiformis, psoriasis; said inflammation of the respiratory tract is selected from pharyngitis, tonsillitis, laryngitis, tracheitis, rhinitis, and said inflammation of the mucous membranes is selected from gingivitis, mouth ulcers and otitis.

3. The association according to claim 1 or 2, wherein said microorganism is the *Lactobacillus reuteri* LMG P-27481 strain deposited at the BCCM/LMG Bacteria Collection microorganism collection centre of the University of Ghent (Belgium).

4. The association according to any one of claims 1 to 3, wherein said microorganism is a live or heat inactivated microorganism.

5. An association comprising a microorganism of the Lactobacillus reuteri species, and resveratrol or a derivative thereof, wherein said microorganism is the Lactobacillus reuteri LMG P-27481 strain deposited at the BCCM/LMG Bacteria Collection microorganism collection centre of the University of Ghent (Belgium), and said derivative of resveratrol is selected from Piceid, Pterostilbene, Trimethoxystilbene, Triacetyl-resveratrol, Tetramethoxysilbene, Pentamethoxystilbene, Dihydroxystilbene, Tetrahydroxystilbene, Hexahydroxystilbene, 2,6-dibromo-4-(3,5-dibromostyryl)phenol, 3,5-di-fluoro-4'-acetoxystilbene, 3,4,5-trimethoxy-4'-bromo-cis-stilbene, 4'bromo-resveratrol, 2-bromo-resveratrol or 2-chloro-resveratrol.

6. The association comprising a microorganism as described in any one of claims 1 to 5, and a plant extract comprising resveratrol, preferably said resveratrol is included in an extract of *Polygonum cuspidatum,* grapes, blueberries or peanuts.

7. The composition comprising the association according to any one of claims 1 to 6, further comprising at least a pharmaceutically and/or nutraceutically acceptable excipient and/or carrier and optionally at least a stabilizing agent, preferably carboxymethyl beta-glucan (CMG) and/or deep eutectic solvents (DES - Deep Eutectic Solvents).

8. The composition according to claim 7, wherein said microorganism is present in a concentration from 10² CFU/g to 10¹² CFU/g, preferably from 10⁶ CFU/g to 10¹⁰ CFU/g, and/or wherein said resveratrol is present in a concentration from 0.0001 % to 50%, preferably from 0.5% to 5% w/v.

9. The composition according to any one of claims 7 or 8, for oral, nasal, aerosol, sublingual, systemic, topical administration.

10. The composition according to any one of claims 7 to 9, in form of suspension, emulsion, cream, ointment, gel, spray, granulate, powder, solution, capsule, tablet.

11. A food supplement or a medical device or a cosmetic product or a pharmaceutical composition or a functional food comprising the association according to any one of claims 1 to 6.

12. The association according to claim 5 or 6, or the composition according to any one of claims 7 to 10, or food supplement, medical device, cosmetic product, pharmaceutical composition or functional food according to claim 11, for use as medicament.

13. The association or composition or food supplement or medical device or cosmetic product or pharmaceutical composition or functional food for use according to claim 12, for use in the prevention, in the treatment or in adjuvating the treatment of an inflammatory disease or an inflammatory state due to viral or bacterial infections.

14. The association or composition or food supplement or medical device or cosmetic product or pharmaceutical composition or functional food for use according to any one of claims 12 or 13, wherein said inflammatory disease or inflammatory state is a dermatological inflammation, an inflammation of the gastrointestinal tract, an inflammation of the mucous membranes or an inflammation of the respiratory tract, preferably wherein said dermatological inflammation is selected from acne, rosacea, atopic dermatitis, contact dermatitis, seborrheic dermatitis, dermatitis eczematous, dermatitis herpetiformis, psoriasis; said inflammation of the gastrointestinal tract is selected from fissures, haemorrhoids, proctitis; said inflammation of the respiratory tract is selected from pharyngitis, tonsillitis, laryngitis, tracheitis, rhinitis, and said inflammation of the mucous membranes is selected from gingivitis, mouth ulcers and otitis.

15. A kit of parts comprising a microorganism of the *Lactobacillus reuteri* species and resveratrol or a derivative thereof, wherein said microorganism is the Lactobacillus reuteri LMG P-27481 strain deposited at the BCCM/LMG Bacteria Collection microorganism collection centre of the University of Ghent (Belgium) and wherein said derivative of resveratrol is selected from Piceid, Pterostilbene, Trimethoxystilbene, Triacetyl-resveratrol, Tetramethoxysilbene, Pentamethoxystilbene, Dihydroxystilbene, Tetrahydroxystilbene, Hexahydroxystilbene, 2,6-dibromo-4-(3,5-dibromostyryl)phenol, 3,5-di-fluoro-4'-acetoxystilbene, 3,4,5-trimethoxy-4'-bromo-cis-stilbene, 4'bromo-resveratrol, 2-bromo-resveratrol or 2-chloro-resveratrol.
